# EUROPEAN PATENT APPLICATION

(11) **EP 3 173 028 A1**
(43) Date of publication of application: **31.05.2017**
(21) Application number: 16198375.4
(22) Date of filing: 11.11.2016
(51) Int. Cl.: A61B 17/00, A61B 17/12, A61F 2/24

(54) **DELIVERY APPARATUS FOR A MEDICAL DEVICE**

(30) Priority: 24.11.2015 TW 104139012; 07.06.2016 TW 105118009
(71) Applicant: Hu, Bobby, Taichung (TW)
(72) Inventor: Hu, Bobby, Taichung (TW)
(74) Representative: Viering, Jentschura & Partner mbB Patent- und Rechtsanwälte

(57) **Abstract**

A delivery apparatus (10; 10a) for delivering a medical device (70) to a heart defect by a catheter (90) includes a second wire section (12; 12a; 12b; 12c) having a first end (1201) securely connected to a first wire section (11). A connecting member (121) is mounted to a second end (1202) of the second wire section (12; 12a; 12b; 12c) and is connected to the medical device (70). The second wire section (12; 12a; 12b; 12c) has a cross sectional area smaller than the first wire section (11). When the medical device (70) is delivered to the heart defect and is seated in a deployed position occluding the heart defect after the catheter (90) is retracted, the second wire section (12; 12a; 12b; 12c) remains connected to the medical device (70) to absorb interfering vibration from the heartbeat, avoiding the medical device (70) from deviating from the deployed position.

## Description

### BACKGROUND OF THE INVENTION

The present invention relates to a delivery apparatus and, more particularly, to a delivery apparatus for a medical device, such as a septal defect occluder.

As its name implies, a congenital heart defect is a problem in the structure of the heart that is present at birth. Namely, the heart of the fetus grows abnormally due to obstruction. According to the statistics, congenital heart defects occur in 7 to 10 per 1000 live births, most of them are ventricular septal defects (VSD), atrial septal defects (ASD) are the second most, and then followed by patent ductus arteriosus (PDA), tetralogy of the Fallot (TOF), pulmonary stenosis (PS), and transposition of the great vessels. There are many types of congenital heart defects, the above defects are merely common ones. Furthermore, a congenital heart defect may occur with two, three, or four abnormal symptoms and, in this, case, is generally referred to as a complex congenital heart defect.

With improvements in the medical therapy, treatment of congenital heart defects has been evolved from open heart surgery to cardiac catheterization (non-cardiac surgery). Current cardiac catheterization includes balloon angioplasty or intravascular stent placement surgery, closure of patent ductus arteriosus, and closure of atrial or ventricular septal defect.

In 1967, Porstmann et al. reported the use of Ivalon plug to close patent ductus arteriosus (PDA), which set a precedent of interventional treatment with a high success rate and with less risk of embolism for curing congenital heart defects. However, this treatment could only be used on children older than 5 years of age. Then, Rashkind and Cuaso developed single and double-umbrella devices for closure of closure of patent ductus arteriosus, and this method was frequently used from the 1970s through the early 1990s. However, this method incurred a high percentage of residual shunting (more than 20%), and, thus, required a second or even a third closure. In 1976, King and Miller for the first time successfully used double-umbrella interventional treatment on central type atrial septal defects, broadening the scope of interventional treatment of congenital heart diseases.

In 1982, Kan first reported five cases of simple pulmonary stenosis cured by percutaneous balloon pulmonary valvuloplasty (PBPV) using a mechanical force of inflation of a balloon to separate the fused commissures at the pulmonary valve leaflets, thereby relieving stenosis of the valve area. This approach includes polyethylene balloon method and Inoue balloon method according to the different balloons used. In 1987 and 1988, Lababidi et al. twice reported success of balloon valvuloplasty on membranous aortic stenosis and congential aortic stenosis, which was the start of balloon valvuloplasty. PBPV has become the internationally recognized first-choice method for congenital cardiac diseases. In 1987, Lock et al. successfully applied Rashkind double-umbrella occluders on vertrycular septal defects that could not be cured by surgery, which made a beneficial exploration in development of interventional treatment of congenital cardiac diseases. In 1992, Combier at al. first used a spring coil to successfully close patent ductus arteriosus. However, the above interventional treatments have various limitations, particularly in occlusion of cardiovascular diseases, such as small applications in diseases, more complications, complicated operation, low success rate, and more residual shunts, adversely affecting clinical popularization. Until 1997, Amplatzer, a U.S. interventional radiologist, invented nickel-titanium shape memory alloy filaments to produce occluders having a shape of a mushroom, a cork, or dual discs, which not only overcomes major disadvantages of conventional interventional treatments but provides advantages including operation in the venous system, small wound, easier operation, wider applications, and obvious theraputic effects.

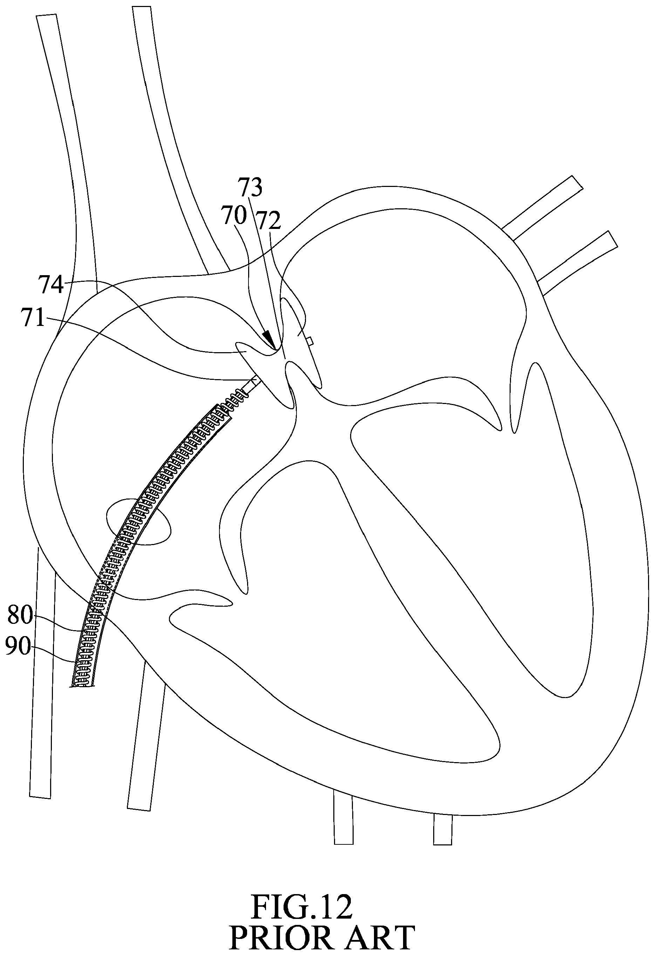

However, current delivery apparatuses for occluders and current delivery procedures still have many disadvantages to be overcome and improved. FIGS. 12-14 show a current delivery apparatus for delivering an occluder 70. A threaded portion 71 of the occluder 70 must firstly be tightly screwed to a distal end of a main control wire 80. The occluder 70 is retracted into a catheter 90 and is inserted into the defect of a patient through the groin. Under guidance of X-ray and ultrasound image, a physician can expand a left atrial disc 72 and a portion of a waist 73 of the occluder 70 according to the hand feel and inspection by the ultrasound image. The occluder 70 can be gently advanced to a position abutting the cardiac septum. The catheter 90 is retraced by tightly pulling the main control wire 80 to expand a right atrial disc 74 of the occluder 70. The catheter 90 is retracted rearward for 5-10 mm. A projection position identical to the angiography is taken to look at the septal defect from side. This can also observed by the ultrasound image to confirm the occluder 70 is properly positioned and the septal defect is stably occluded. Then, the main control wire 80 can be rotated to detach the occluder 70.

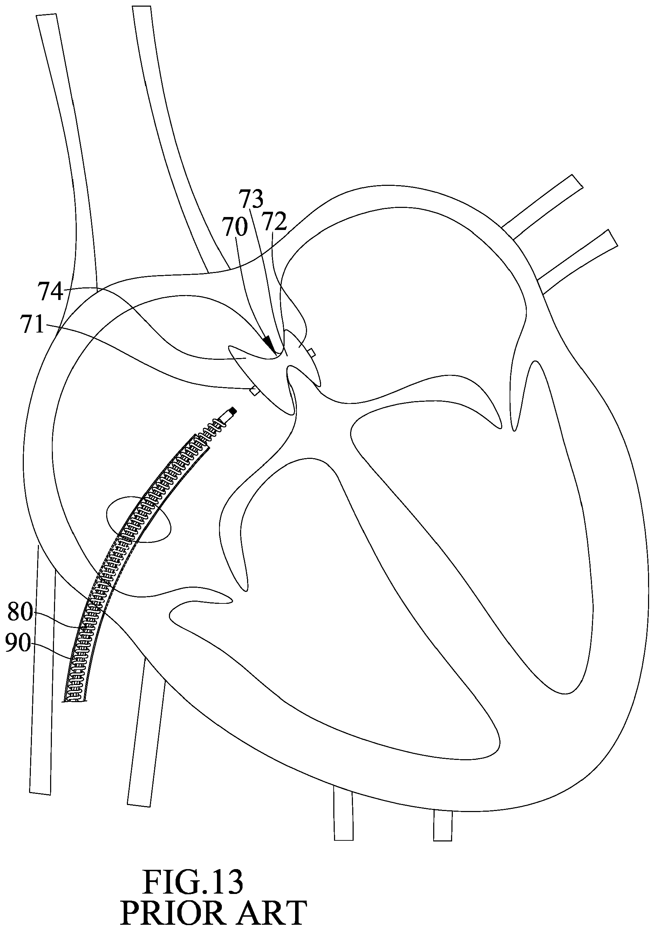

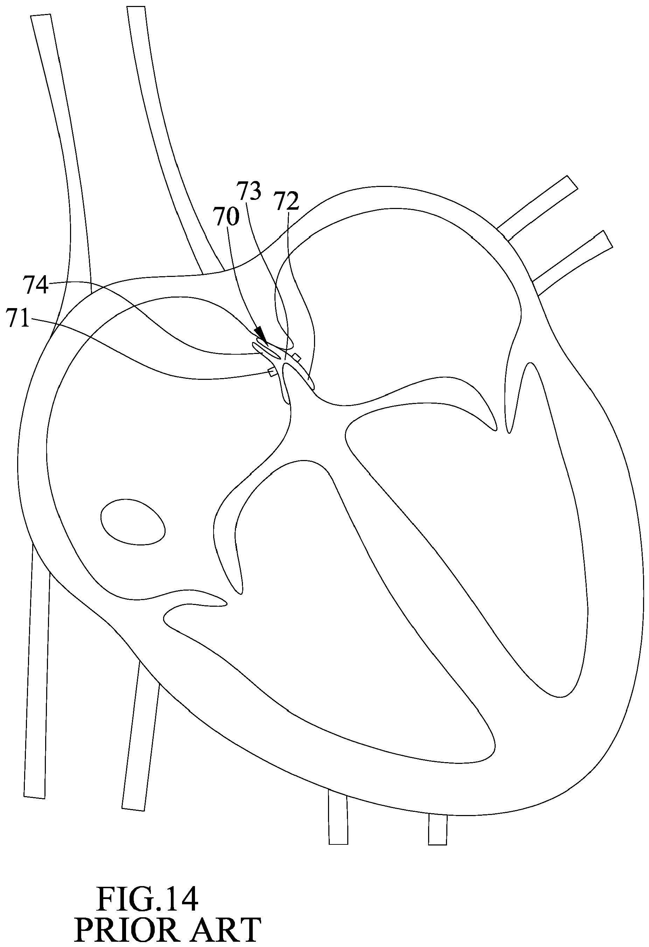

Since the interference of the heartbeat, the blood vessel (FIG. 13 shows an inferior vena cava), and the stiffness of the main control wire 80 limit the operating angle of the catheter 90 inside the heart, the physician can only complete the installation by the hand feel and ultrasound observation during delivery of the occlude 70. When the moment the main control wire 80 is rotated to detach the occluder 70 from the main control wire 80, due to the interference by the heartbeat as well as the tension of the main control wire 80 and the reactive force of the elasticity of the occluder 70 (see FIG. 13) at the moment the occluder 70 is detached from the main control wire 80, the left atrial disc 72 could shrink and deform in the left atrium and move through the septal defect to the right atrium (see FIG. 14), resulting in failure in occluding the septal defect. In this case, a foreign-body forceps and a snare are used to cooperate with the catheter 90 in an attempt to catch the threaded portion 71 of the occluder 70 for retracting the occluder 70 into the catheter 90 and then withdrawing the catheter 90 out of the body. If unsuccessful, emergent surgery is desirable to prevent delay in treatment that may cause serious consequence or even death of the patient. In a case that the occluder 70 gets stuck in the tricuspid valve or the bicuspid valve, timely surgery is desired to remove the occluder 70. Thus, it can be seen that the interference of the heartbeat, the blood vessel, and the stiffness of the main control wire 80 restrict the operating angle of the catheter 90 inside the heart, and at the moment the occluder 70 is detached from the main control wire 80, the tension of the main control wire 80 and the reactive force of the elasticity of the occluder 70 are the main factor causing failure in occluding the septal defect by the occluder 70. Serious consequences could, thus, be incurred.

Furthermore, when the threaded portion 71 of the occluder 70 is tightly screwed to the distal end of the main control wire 80, the physician can only depend on previous experience to judge the locking force between the occluder 70 and the main control wire 80. If too tight, the occluder 70 cannot smoothly be detached from the main control wire 80 after the septal defect is occluded, or a great vibration is caused during detachment, leading to failure in occluding the septal defect. When the occluder 70 advances to the wrong position, the occluder 70 must be retrieved and redeployed and may cause serious consequences.

U.S. Patent No. 6,913,614 discloses a delivery system for a medical device with an occluder. The delivery system includes a hollow delivery device for positioning the medical device via a catheter, and a flexible tether is provided to connect the medical device and the delivery device. After the medical device is seated by the delivery device, the tether is detached from the delivery device but remains connected to the medical device. The tether is fixed to a coupler having a first threaded side for threading connection with the hollow delivery device and a second threaded side for threading connection with the medical device.

The tether design of the above patent solves the improper seating problem of the occluder and can reliably retrieve the occluder, avoiding the risk of emergent surgery. However, the interference from the heartbeat, the blood vessel, and the stiffness of the hollow delivery device restrict the operating angle of the catheter inside the heart. Furthermore, at the moment the occluder is disconnected from the coupler, the reactive force of the elasticity of the occluder results in improper seating of the occluder, and the cause still cannot be solved by now. Furthermore, the outer diameter of the hollow delivery device must be large enough to accommodate the tether design, and a catheter with a larger outer diameter must be used, resulting in great discomfort to the patient when the catheter is passing through the blood vessel of the patient. Furthermore, the operating angle of the catheter with an outer diameter is more apt to be affected by the blood vessel, causing difficulties in delivery of the occluder inside the heart. As a result, more problems are incurred.

### BRIEF SUMMARY OF THE INVENTION

A delivery apparatus for a medical device according to the present invention is configured to deliver a medical device to a heart defect in a heart by a catheter. The delivery apparatus includes a first wire section having a proximal end and a distal end and a second wire section having a first end and a second end opposite to the first end. The first end of the second wire section is securely connected to the distal end of the first wire section. A connecting member is mounted to the second end of the second wire section and is adapted to be connected to the medical device. The second wire section has a cross sectional area smaller than a cross sectional area of the first wire section. When the medical device is delivered by the delivery apparatus to the heart defect and is seated in a deployed position occluding the heart defect after the catheter is retracted, the second wire section is configured to have a flexibility maintaining connection with the medical device to absorb interfering vibration from the heartbeat, avoiding the medical device from deviating from the deployed position.

In an example, the delivery apparatus further includes a coupling member between the first wire section and the second wire section. The coupling member is riveted to the first wire section and the second wire section to securely connect the first end of the second wire section to the distal end of the first wire section.

In an example, the first wire section is formed by a plurality of metal wires stranded together. The second wire section is formed by at least one of the plurality of metal wires. The number of the at least one of the plurality of metal wires forming the second wire section is smaller than the number of the plurality of metal wires forming the first wire section.

In an example, the second wire section is formed by one of the plurality of metal wires.

In an example, a virtual axis extends through an axis of the first wire section and an axis of the second wire section. The first wire section has a maximum width perpendicular to the virtual axis. The second wire section has a maximum width perpendicular to the virtual axis. The maximum width of the second wire section is not larger than the maximum width of the first wire section. A coupling member is mounted between the first wire section and the second wire section. The coupling member is riveted to the first wire section and the second wire section to securely connect the second wire section to the first wire section.

In an example, the connecting member includes a threaded portion in threading connection with the medical device. The threaded portion has a twist controllability configured to permit the threaded portion to rotate relative to the medical device when the delivery apparatus is rotated, thereby releasing the medical device.

In an example, the first wire section is formed by a plurality of metal wires stranded together in a rotational direction, and the threaded portion has a thread direction in the rotational direction.

In an example, the second wire section has a length permitting the second wire section to be completely located in the heart without contacting with blood vessels of the heart after the medical device has been deployed to occlude the heart defect and after the catheter has been retracted.

In an example, the second wire section has circular cross sections, a a spring wire is coiled around the first wire section, and the medical device is a heart defect occluder.

In another example, the second wire section has non-circular cross sections, a spring wire is coiled around the first wire section, and the medical device is a heart defect occluder.

In an example, a virtual axis extends through an axis of the first wire section and an axis of the second wire section. The second wire section includes a flexible portion capable of absorbing interfering vibration from the heartbeat. The flexible portion includes a first portion and a second portion. Each of the first portion and the second portion extends along the virtual axis. The first portion has a first end securely connected to the distal end of the first wire section. The second wire section has a first end connected to a second end of the first portion opposite to the first wire section.

In an example, the first portion has a uniform cross sectional area perpendicular to the virtual axis and equal to a maximum cross sectional area of the flexible portion of the second wire section, and the second portion has a uniform cross sectional area smaller than the uniform cross sectional area of the first portion.

In an example, the flexible portion of the second wire section has a maximum width perpendicular to the virtual axis. The first portion has a maximum width perpendicular to the virtual axis. The maximum width of the first portion is equal to the maximum width of the flexible portion of the second wire section. The second portion has a maximum width perpendicular to the virtual axis. The maximum width of the second portion is not larger than the maximum width of the first portion.

In an example, the delivery apparatus further includes a first coupler between the first portion and the second portion. The first coupler includes a first end riveted to the second end of the first portion and a second end riveted to the second portion. Riveting of the first coupler includes:
forming two recessed portions in two diametrically opposed sides of the second end of the first portion by extrusion;
forming two first recessed portions in two diametrically opposed sides of the first end of the second portion by extrusion; and
mounting the first coupler around the second end of the first portion and the first end of the second portion, with the first coupler including a first end and a second end, with two diametrically opposed sides of the first end of the first coupler being punched to form two first recessed portions, with two diametrically opposed sides of the second end of the first coupler being punched to form two second recessed portions, with the two first recessed portions of the first coupler received in and aligned with the two recessed portions of the first portion, and with the two second recessed portions of the first coupler received in and aligned with the two first recessed portions of the second portion.

In an example, the flexible portion of the second wire section further includes a third portion extending along the virtual axis. The third portion includes a first end connected to a second end of the second portion opposite to the first portion. The third portion has a cross sectional area smaller than a cross sectional area of the second portion. The third portion has a maximum width perpendicular to the virtual axis. The maximum width of the third portion is not larger than the maximum width of the second portion. A second coupler is mounted between the second portion and the third portion. The second coupler includes a first end riveted to the second portion and a second end riveted to the third portion. Riveting of the second coupler includes:
forming two second recessed portions in two diametrically opposed sides of the second end of the second portion by extrusion;
forming two first recessed portions in two diametrically opposed sides of the first end of the third portion by extrusion; and
mounting the second coupler around the second end of the second portion and the first end of the third portion, with the second coupler including a first end and a second end, with two diametrically opposed sides of the first end of the second coupler being punched to form two first recessed portions, with two diametrically opposed sides of the second end of the second coupler being punched to form two second recessed portions, with the two first recessed portions of the second coupler received in and aligned with the two second recessed portions of the second portion, and with the two second recessed portions of the second coupler received in and aligned with the two first recessed portions of the third portion.

In an example, the first coupler includes a first engaging member and a second engaging member. The two first recessed portions of the first coupler are formed in the first engaging member of the first coupler. The first engaging member of the first coupler is securely connected to the second end of the first portion. The two second recessed portions of the first coupler are formed in the second engaging member of the first coupler. The second engaging member of the first coupler is securely connected to the first end of the second portion. The first and second engaging members of the first coupler are fixed to each other by screwing and bonding. The second coupler includes a first engaging member and a second engaging member. The two first recessed portions of the second coupler are formed in the first engaging member of the second coupler. The first engaging member of the second coupler is securely connected to the second end of the second portion. The two second recessed portions of the second coupler are formed in the second engaging member of the second coupler. The second engaging member of the second coupler is securely connected to the first end of the third portion. The first and second engaging members of the second coupler are fixed to each other by screwing and bonding.

In an example, the connecting member is riveted to a second end of the third portion opposite to the second portion. Riveting of the connecting member includes:
forming two second recessed portions in two diametrically opposed sides of the second end of the third portion by extrusion; and
mounting the connecting member around the second end of the third portion, with two diametrically opposed sides of the connecting member being punched to form two first recessed portions, and with the two first recessed portions of the connecting member received in and aligned with the two second recessed portions of the third portion.

In an example, each of the first coupler and the second coupler is integrally formed.

The present invention will become clearer in light of the following detailed description of illustrative embodiments of this invention described in connection with the drawings.

### DESCRIPTION OF THE DRAWINGS

FIG. 1 is a partial, perspective view of a delivery apparatus for a delivery device of a first embodiment according to the present invention.
FIG. 2 is a partial, cross sectional view of the delivery device of FIG. 1.
FIG. 2A is a cross sectional view taken along section line 2A-2A of FIG. 2.
FIG. 2B is an enlarged view of a circled portion of FIG. 2.
FIG. 3 is a schematic diagram illustrating use of the delivery apparatus of FIG. 1 with a catheter withdrawn to expand a left atrial disc of an occluder.
FIG. 4 is a view similar to FIG. 3, with a second wire section remained attached to the occlude to absorb interfering vibration caused by the heartbeat, and with the catheter further withdrawn to expand the right atrial disc for occluding the atrial septal defect.
FIG 5 is a view similar to FIG. 4, with the occluder seated properly and reliably occluding the septal defect, and with a first wire section rotated to release the occluder.
FIG. 6 is a partial, cross sectional view of a delivery apparatus of a second embodiment according to the present invention.
FIG. 6A is a cross sectional view taken along section line 6A-6A of FIG. 6.
FIG. 7 is a partial, plan view of a delivery apparatus of a third embodiment according to the present invention.
FIG. 8 is an exploded, perspective view of the delivery apparatus of FIG 7.
FIG. 9 is a partial, cross sectional view of the delivery apparatus of FIG. 7.
FIG. 9A is a cross sectional view taken along section line 9A-9A of FIG. 9.
FIG. 9B is a schematic cross sectional view of the delivery apparatus of FIG. 7 before assembly.
FIG. 10 is a perspective view of a delivery apparatus of a fourth embodiment according to the present invention.
FIG. 11 is an exploded, perspective view of the delivery device of FIG. 10.
FIG. 12 is a diagrammatic view illustrating the deployment of a current occluder at a cardiac defect after a delivery procedure.
FIG. 13 is a view similar to FIG. 12, with the occluder detached from a main control wire.
FIG 14 is a view similar to FIG. 13, with a left atrial disc of the occluder deformed and moved through the septal defect to the right atrium.

### DETAILED DESCRIPTION OF THE INVENTION

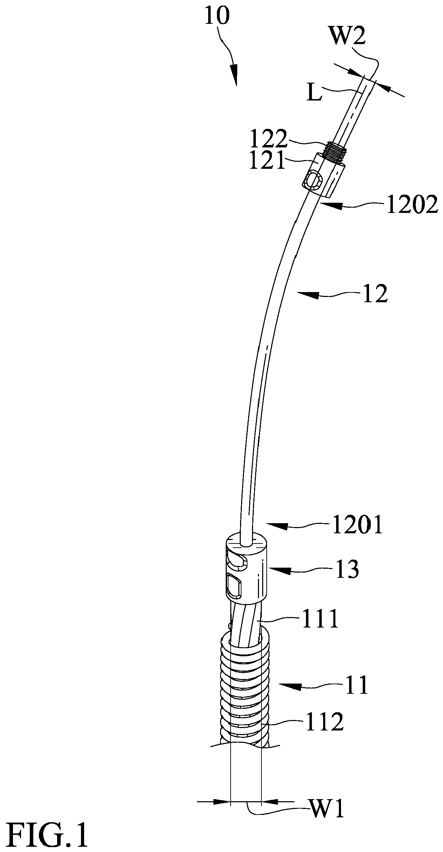

FIGS. 1-5 show a delivery apparatus 10 of a first embodiment according to the present invention. The delivery apparatus 10 is configured to deliver a medical device 70 to a heart defect in a heart by a catheter 90. In this embodiment, the heart defect is an atrial septal defect, and the medical device 70 is a septal defect occluder (hereinafter occluder 70), such as Amplatzer septal occluder. Occluder 70 is obtained by weaving nickel-titanium shape memory alloy into a reticulate metal structure and has spontaneous expansion properties. In this embodiment, occluder 70 includes an end having a threaded portion 71 for connection with delivery apparatus 10. Threaded portion 71 is a nut including a central hole having an inner thread. Furthermore, occluder 70 includes left and right atrial discs 72 and 74 having spontaneous expansion properties and a waist 73 between left and right atrial discs 72 and 74.

Delivery apparatus 10 includes a first wire section 11 and a second wire section 12. First wire section 11 has a proximal end (not shown) and a distal end.

Second wire section 12 includes a first end 1201 and a second end 1202 opposite to first end 1201. First end 1201 of second wire section 12 is securely connected to the distal end of first wire section 11. A connecting member 121 is mounted to second end 1202 of second wire section 12 and is adapted to be connected to occluder 70. Second wire section 12 has a cross sectional area smaller than a cross sectional area of first wire section 11. When occluder 70 is delivered by delivery apparatus 10 to the heart defect and is seated in a position occluding the heart defect after catheter 90 is retracted, second wire section 12 is configured to have a flexibility maintaining connection with occluder 70 to absorb interfering vibration from the heartbeat. Furthermore, during release of occluder 70, the flexibility of second wire section 12 avoids occlude 70 from deviating from the position. The flexibility of second wire section 12 is associated with the stiffness and elasticity of second wire section 12. The flexibility of second wire section 12 avoids the limitation in the operating angle of catheter 90 inside the heart resulting from the blood vessel, and the stiffness of first wire section 11.

A coupling member 13 is mounted between first wire section 11 and second wire section 12. Coupling member 13 is riveted to first wire section 11 and second wire section 12 to securely connect first end 1201 of second wire section 12 to the distal end of first wire section 11.

A virtual axis L extends through an axis of first wire section 11 and an axis of second wire section 12. First wire section 11 has a maximum width W1 perpendicular to virtual axis L. Second wire section 12 has a maximum width W2 perpendicular to virtual axis L. Maximum width W2 of second wire section 12 is not larger than maximum width W1 of first wire section 11.

Connecting member 121 includes a threaded portion 122 in threading connection with occluder 70. Threaded portion 122 has a twist controllability configured to permit threaded portion 122 to rotate relative to occluder 70 when delivery apparatus 10 is rotated, thereby releasing occluder 70. The twist controllability of second wire section 12 is associated with the stiffness, elasticity, and flexibility of second wire section 12.

Note that the twist controllability of threaded portion 122 permits threaded portion 122 to rotate relative to threaded portion 71 of occluder 70 when the proximal end of first wire section 11 of delivery apparatus 10 is rotated (thereby releasing occluder 70), namely, the flexibility of second wire section 12 is set to withstand the torque resulting from rotating connecting member 121, such that threaded portion 122 disengages from threaded portion 71 of occluder 70. Thus, before practicing occlusion of the heart defect, a physician screwing threaded portion 71 of occluder 70 to threaded portion 122 of second wire section 12 can stop rotating occluder 70 if he or she notices second wire section 12 starts to twist. A suitable locking force is achieved to avoid over locking of occluder 70 that may lead to failure in smooth release of occluder 70 in the heart.

In this embodiment, first wire section 11 is formed by a plurality of metal wires 111 stranded together. A spring wire 112 is coiled around first wire section 11 to increase the stiffness of first wire section 11. Metal wires 111 are stranded in a rotating direction to form first wire section 11, and threaded portion 122 has a thread direction in the rotational direction. The rotating direction can be the clockwise direction or the counterclockwise direction. In this embodiment, the rotating direction is the clockwise direction.

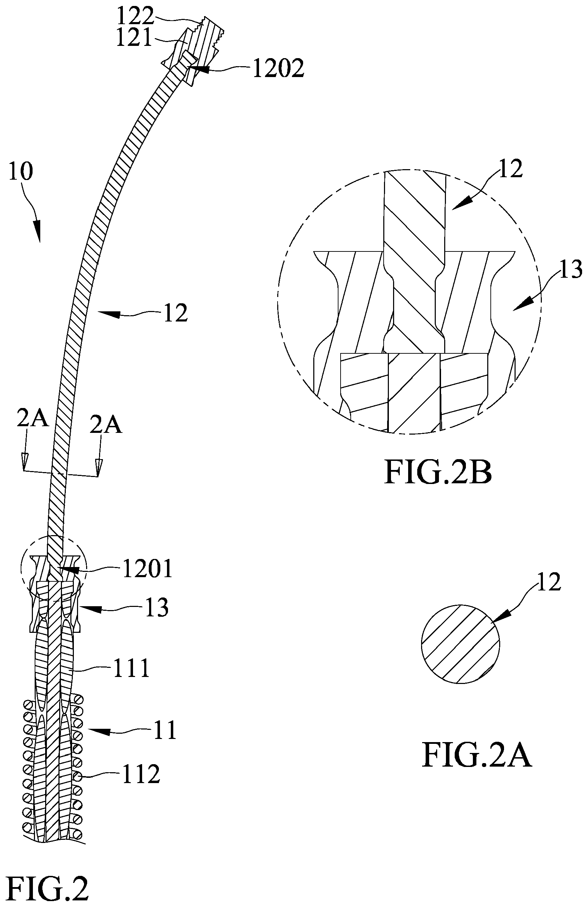

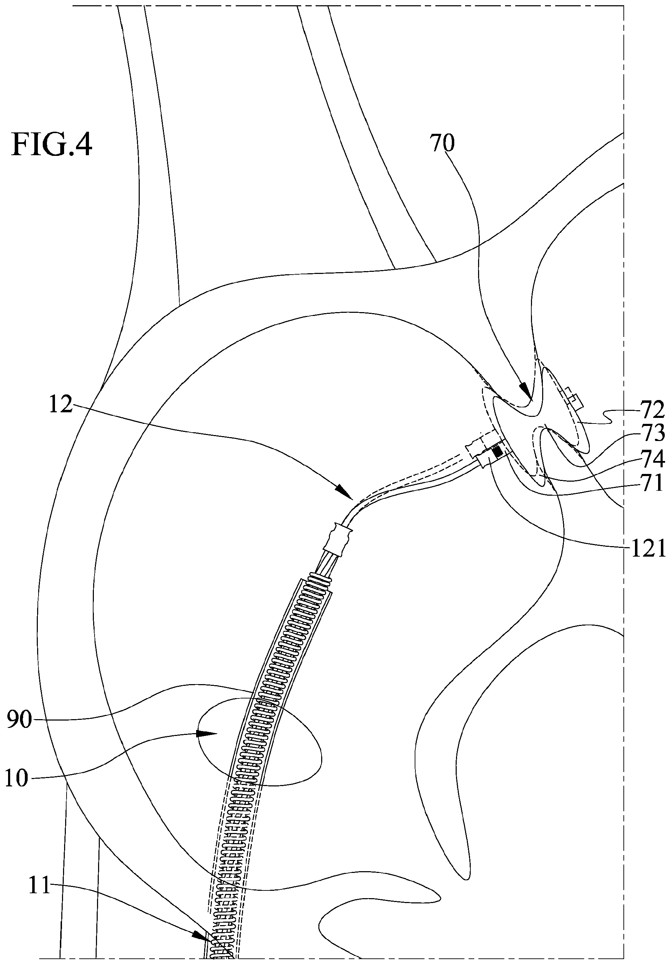

Second wire section 12 has a length to be completely located in the heart without contacting with blood vessels of the heart (see FIG. 4) after occluder 70 has been deployed to occlude the heart defect by operating first wire section 11 and after the catheter 90 has been retracted. Since the size of the heart of a child is different from an adult, the physician can select second wire section 12 of a desired length according to the size of the heart of the patient to proceed with occlusion of the heart defect. For example, a child has a heart smaller than an adult, such that a delivery apparatus 10 having a second wire section 12 of a smaller length can be used to proceed with occlusion of the heart defect in the heart of the child. In this embodiment, second wire section 12 has circular cross sections (FIG. 2A).

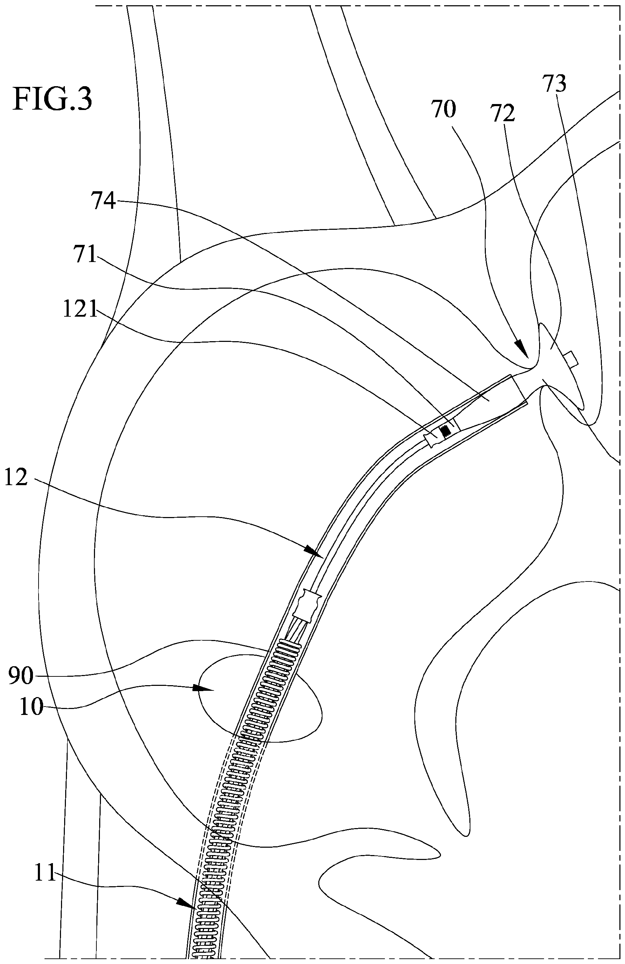

With reference to FIG. 3, physician places first wire section 11 and occluder 70 into catheter 90 and advances occlude 70 to the heart defect of the patient. Then, the physician retracts catheter 90 to expand left atrial disc 72 and a portion of waist 73. At the same time, first wire section 11 is used to pull occluder 70 until left atrial disc 72 of occluder 70 abuts against the cardiac septum. Thus, a side of the heart defect adjacent to the left atrium is closed by left atrial disc 72.

With reference to FIG. 4, the physician then retracts catheter 90 further to expand right atrial disc 74 to close the other side of the heart defect adjacent to the right atrium. In this case, second wire section 12 is completely outside of catheter 90 and remains connected to occluder 70 to absorb interfering vibration resulting from the heartbeat or to absorb impact resulting from the flow of blood in the vessels in the heart.

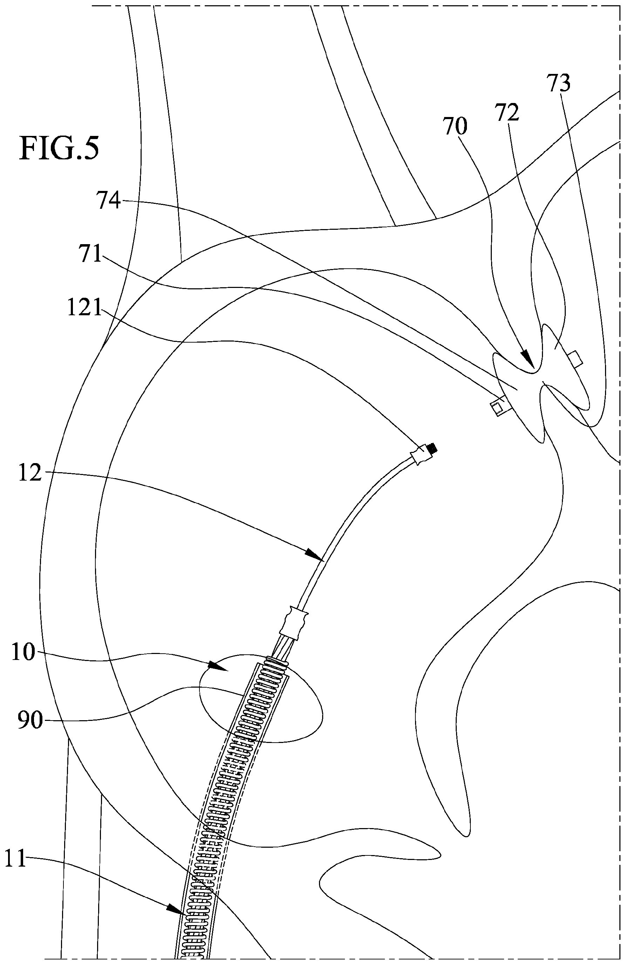

With reference to FIG. 5, after left atrial disc 72 and right atrial disc 74 have expanded and occluded the heart defect, ultrasound observation can be used to confirm whether occluder 70 is seated properly and whether occluder 70 stably occludes the heart defect. If it is confirmed that occluder 70 is seated properly and occluder 70 stably occludes the heart defect, the proximal end of first wire section 11 is rotated to drive connecting member 121 to rotate, such that threaded portion 122 of second wire section 12 rotates relative to threaded portion 71 of occluder 70, thereby releasing occluder 70.

Since the twist controllability of threaded portion 122 permits threaded portion 122 to rotate relative to occluder 70 when second wire section 12 is rotated (thereby releasing occluder 70) and since first wire section 11 is connected to occluder 70 via second wire section 12 (not directly connected to occluder 70), the influence on occluder 70 (namely, shrinkage and deformation of left atrial disc 72 in the left atrium, leading to disengagement from the heart defect, or deviation of occluder 70 from the proper position) resulting from the tension of first wire section 11 and the reactive force of the elasticity of occluder 70 can be avoided.

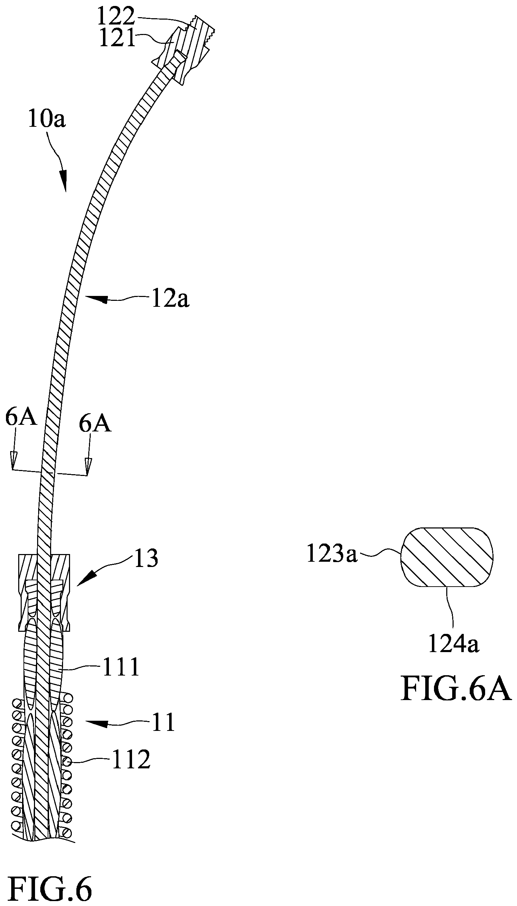

FIG. 6 shows a delivery apparatus 10a of a second embodiment according to the present invention substantially the same as the first embodiment. The second embodiment is different from the first embodiment by that second wire section 12a is formed by at least one of metal wires 111. Namely, a portion of at least one of metal wires 111 forming second wire section 12a and the remaining metal wires 111 are stranded together to form first wire section 11. The number of the at least one of the metal wires 111 forming second wire section 12a is smaller than the number of metal wires 111 forming first wire section 11. In this embodiment, second wire section 12a is formed by one of metal wires 111.

In this embodiment, second wire section 12a has non-circular cross sections perpendicular to virtual axis L. Second wire section 12a includes two arcuate sides 123a opposite to each other and two rectilinear sides 124a opposite to each other. Thus, the cross sectional shape of second wire section 12a forms an oval (FIG. 6A). Second wire section 12a and coupling member 13 can be easily riveted together by applying force to rectilinear sides 124a, thereby increasing the engaging strength after riveting and increasing stability.

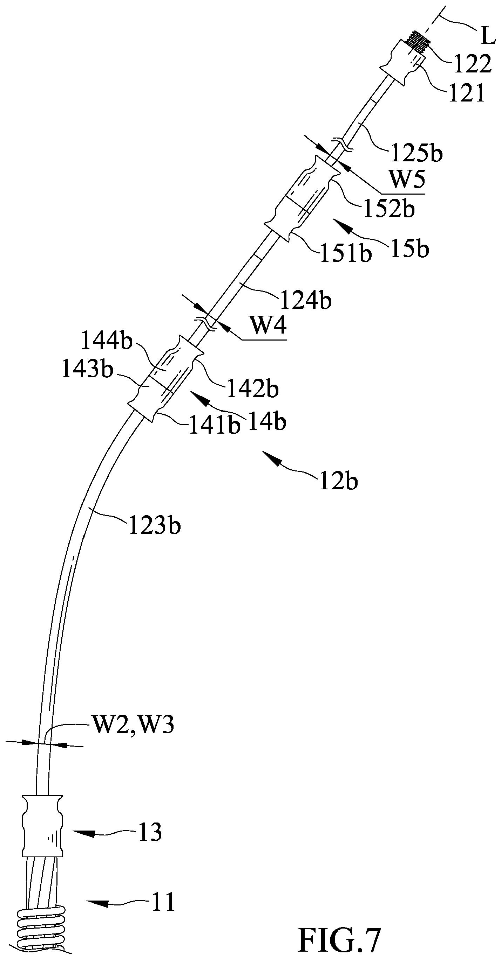

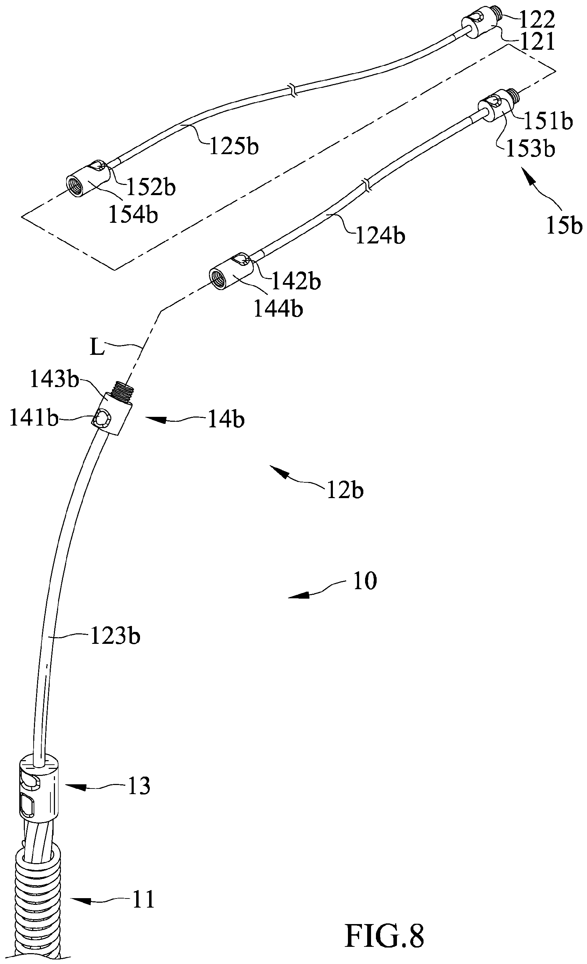

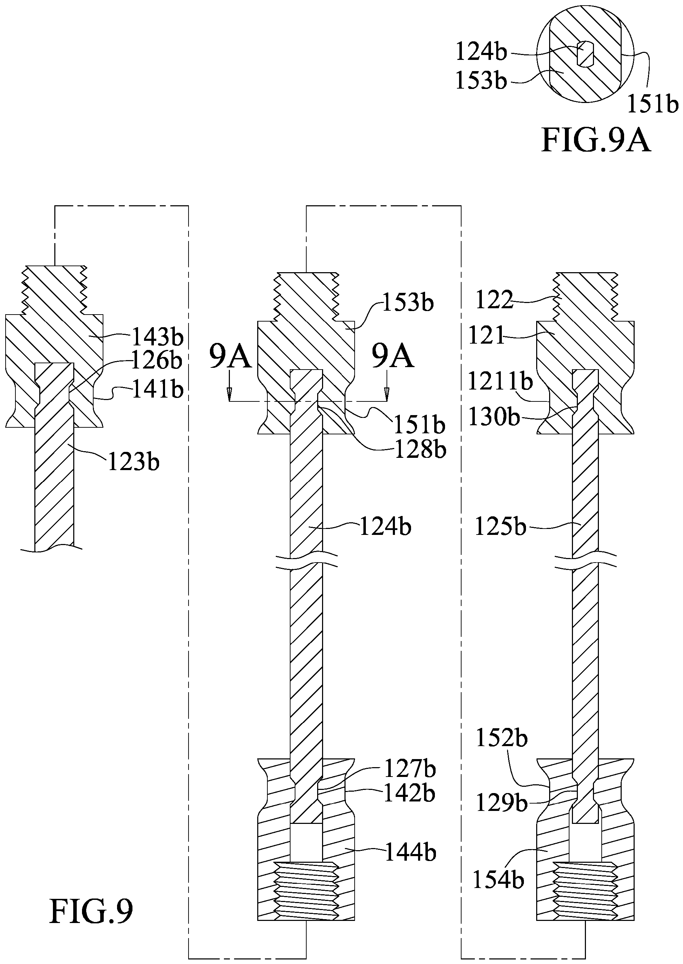

FIGS. 7-9 and 9A show a delivery apparatus 10 of a third embodiment according to the present invention substantially the same as the first embodiment. The third embodiment is different from the first embodiment by that second wire section 12b includes a flexible portion capable of absorbing interfering vibration from the heartbeat. The flexible portion includes a first portion 123b and a second portion 124b. Each of first portion 123b and second portion 124b extends along virtual axis L. First portion 123b has a uniform cross sectional area that is perpendicular to virtual axis L and that is equal to the maximum cross sectional area of the flexible portion of second wire section 12b. Second portion 124b has a uniform cross sectional area smaller than the uniform cross sectional area of first portion 123b. First portion 123b has a first end securely connected to the distal end of first wire section 11. Second wire section 12b has a first end connected to a second end of first portion 123b opposite to first wire section 11. First portion 123b has a maximum width W3 that is perpendicular to virtual axis L and that is equal to maximum width W2 of the flexible portion of second wire section 12b. Second portion 124b has a maximum width W4 that is perpendicular to virtual axis L and that is not larger than the maximum width W3 of first portion 123b.

The flexible portion of second wire section 12b further includes a third portion 125b extending along virtual axis L. Third portion 125b has a uniform cross sectional area perpendicular to virtual axis L. Third portion 125b includes a first end connected to a second end of second portion 124b opposite to first portion 123b. Connecting member 121 can be riveted to a second end of third portion 125b opposite to second portion 124b and can be connected to occluder 70 (see FIGS. 3-5). Third portion 125b has a cross sectional area smaller than the cross sectional area of second portion 124b. Third portion 125b has a maximum width W5 that is perpendicular to virtual axis L and that is not larger than maximum width W4 of second portion 124b.

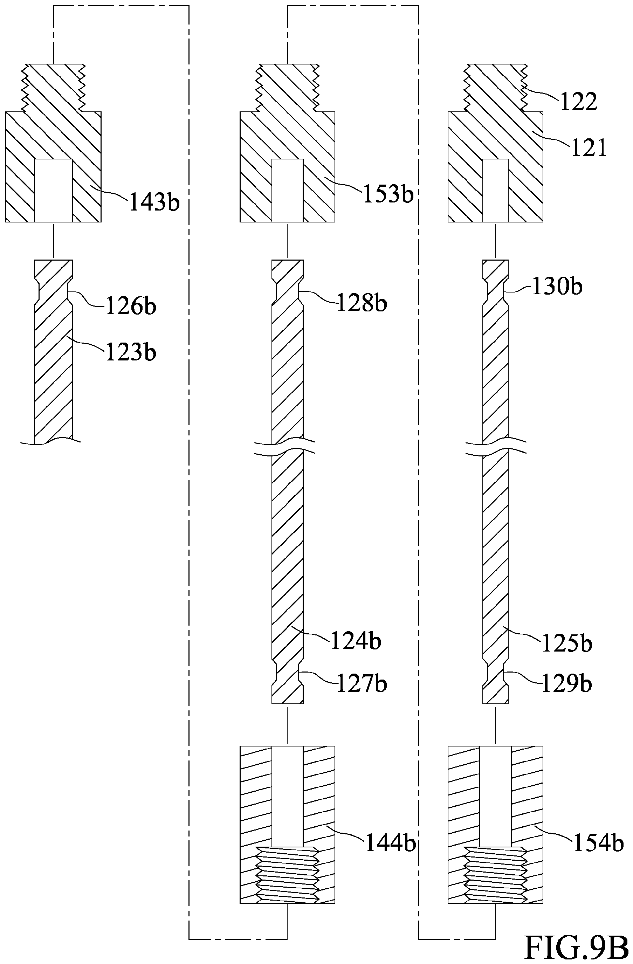

FIG. 9B shows the delivery apparatus of the third embodiment before assembly. A first coupler 14b is mounted between first portion 123b and second portion 124b. First coupler 14b includes a first end riveted to the second end of first portion 123b and a second end riveted to second portion 124b. Riveting of first coupler 14b includes forming two recessed portions 126b in two diametrically opposed sides of the second end of first portion 123b by extrusion. Two first recessed portions 127b are formed in two diametrically opposed sides of the first end of the second portion 124b by extrusion. Next, first coupler 14b is around the second end of first portion 123b and the first end of second portion 124b. First coupler 14b includes a first end and a second end. Two diametrically opposed sides of the first end of first coupler 14b are punched to form two first recessed portions 141b. Two diametrically opposed sides of the second end of first coupler 14b are punched to form two second recessed portions 142b. First recessed portions 141b of first coupler 14b are received in and aligned with recessed portions 126b of first portion 123b. Second recessed portions 142b of first coupler 14b are received in and aligned with first recessed portions 127b of second portion 124b. By the above riveting method, first coupler 14b can be easily and quickly coupled in first portion 123b and second portion 124b. The connection is very reliable while avoiding relative rotation between first coupler 14b, first portion 123b, and second portion 124b.

First coupler 14b includes a first engaging member 143b and a second engaging member 144b. The two first recessed portions 141b of first coupler 14b are formed in first engaging member 143b of first coupler 14b. First engaging member 143b of first coupler 14b is securely connected to the second end of first portion 123b. The two second recessed portions 142b of first coupler 14b are formed in second engaging member 144b of first coupler 14b. Second engaging member 144b of first coupler 14b is securely connected to the first end of second portion 124b. First and second engaging members 143b and 144b of first coupler 14b are fixed to each other by screwing and bonding, and the thread direction of first coupler 14b is opposite to that of threaded portion 122 of connecting member 121.

A second coupler 15b is mounted between second portion 124b and third portion 125b. Second coupler 15b includes a first end riveted to second portion 124b and a second end riveted to third portion 125b. Riveting of second coupler 15b includes forming two second recessed portions 128b in two diametrically opposed sides of the second end of the second portion 124b by extrusion. Two first recessed portions 129b are formed in two diametrically opposed sides of the first end of the third portion 125b by extrusion. Then, second coupler 15b is around the second end of second portion 124b and the first end of third portion 125b. Second coupler 15b includes a first end and a second end. Two diametrically opposed sides of the first end of second coupler 15b are punched to form two first recessed portions 151b. Two diametrically opposed sides of the second end of second coupler 15b are punched to form two second recessed portions 152b. First recessed portions 151b of second coupler 15b are received in and aligned with second recessed portions 128b of the second portion 124b. Second recessed portions 152b of second coupler 15b are received in and aligned with first recessed portions 129b of third portion 125b. Connecting member 121 is riveted to the second end of third portion 125b opposite to second portion 124b. Riveting of the connecting member 121 includes forming two second recessed portions 130b in two diametrically opposed sides of the second end of third portion 125b by extrusion. Connecting member 121 is mounted around the second end of third portion 125b. Two diametrically opposed sides of connecting member 121 are punched to form two first recessed portions 1211. First recessed portions 1211 of connecting member 121 are received in and aligned with second recessed portions 130b of third portion 125b. Coupling member 13 in the first, second, and third embodiments can be riveted by the above riveting method and will not be described in detail to avoid redundancy.

Second coupler 15b includes a first engaging member 153b and a second engaging member 154b. The two first recessed portions 151b of second coupler 15b are formed in first engaging member 153b of second coupler 15b. First engaging member 153b of second coupler 15b is securely connected to the second end of second portion 124b. The two second recessed portions 152b of second coupler 15b are formed in second engaging member 154b of second coupler 15b. Second engaging member 154b of second coupler 15b is securely connected to the first end of third portion 125b. First and second engaging members 153b and 154b of second coupler 15b are fixed to each other by screwing and bonding, and the thread direction of second coupler 15b is opposite to that of threaded portion 122 of connecting member 121.

By providing a second wire section 12b having a plurality of portions, a manufacturer can produce flexible wires having first, second, and third portions 123b, 124b, and 125b with different cross sectional areas and different lengths. In a case that the manufacturer intends to produce second wire sections 12b of different thicknesses and different lengths according to different heart sizes of different patients varied in sizes and ages, various combinations of first portion 123b, second portion 124b, and third portion 125b can be achieved (two or more of them can be connected). Thus, second wire section 12b can be easily and rapidly mass-produced by modularization. Furthermore, second wire section 12b including a head end and a tail end having a cross sectional area smaller than a cross sectional area of the head end can be produced, such that the head end of second wire section 12b is more stiff than the tail end and is easier to operate while the tail end of second wire section 12b is more flexible and has a larger operating angle.

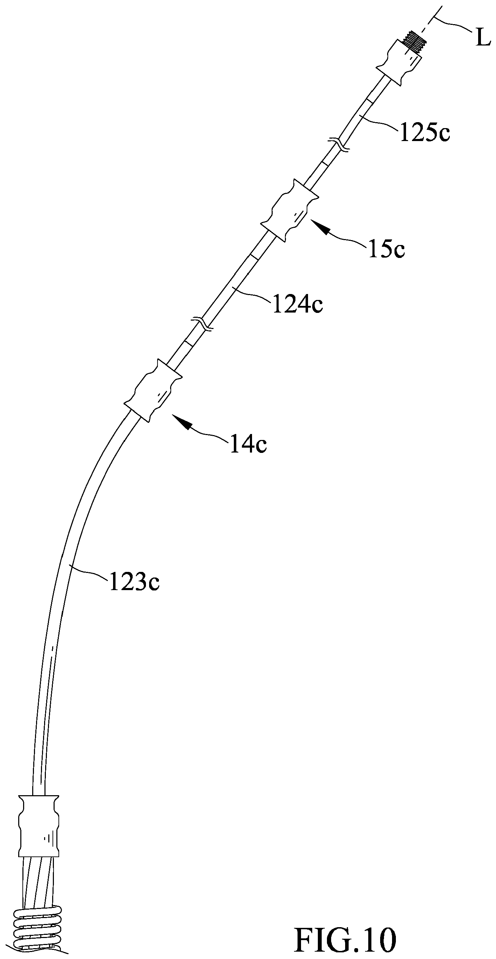

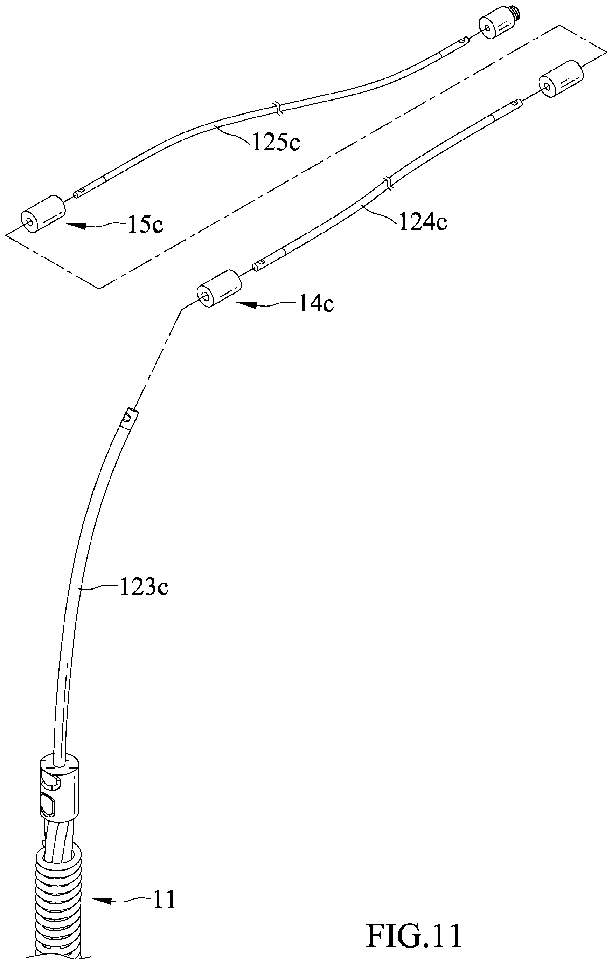

FIGS. 10 and 11 show a delivery apparatus of a fourth embodiment according to the present invention substantially the same as the third embodiment. The fourth embodiment is different from the third embodiment by that each of first coupler 14c and second coupler 15c is integrally formed. Two ends of first coupler 14c are securely connected to first portion 123c and second portion 124c. Two ends of second coupler 15c are securely connected to second portion 124c and third portion 125c.

In view of the foregoing, delivery apparatus 10, 10a according to the present invention includes the following advantages:
1. When occluder 70 is delivered by delivery apparatus 10 to the heart defect and is seated in a deployed position occluding the heart defect after catheter 90 is retracted, second wire section 12, 12a, 12b, 12c is configured to have a flexibility maintaining connection with occluder 70 to absorb interfering vibration from the heartbeat. Furthermore, during release of occluder 70, the flexibility of second wire section 12, 12a, 12b, 12c avoids occluder 70 from deviating from the deployed position.
2. The flexibility of second wire section 12, 12a, 12b, 12c avoids limitation of the operating angle inside the heart due to the blood vessels and the stiffness of first wire section 11.
3. The twist controllability of threaded portion 122 permits threaded portion 122 to rotate relative to threaded portion 71 of occluder 70 when the proximal end of first wire section 11 of delivery apparatus 10, 10a is rotated (thereby releasing occluder 70), namely, the flexibility of second wire section 12, 12a, 12b, 12c is set to withstand the torque resulting from rotating connecting member 121, such that threaded portion 122 disengages from threaded portion 71 of occluder 70. Thus, before practicing occlusion of the heart defect, a physician screwing threaded portion 71 of occluder 70 to threaded portion 122 of second wire section 12, 12a, 12b, 12c can stop rotating occluder 70 if he or she notices second wire section 12, 12a, 12b, 12c starts to twist. A suitable locking force is achieved to avoid over locking of occluder 70 that may lead to failure in smooth release of occluder 70 in the heart.
4. Second wire section 12, 12a, 12b, 12c has a length to be completely located in the heart without contacting with the blood vessels of the heart after occluder 70 has been deployed to occlude the heart defect by operating first wire section 11 and after the catheter 90 has been retracted. Since the size of the heart of a child is different from an adult, the physician can select second wire section 12, 12a, 12b, 12c of a desired length according to the size of the heart of the patient to proceed with occlusion of the heart defect. For example, a child has a heart smaller than an adult, such that a delivery apparatus 10, 10a having a second wire section 12, 12a, 12b, 12c of a smaller length can be used to proceed with occlusion of the heart defect in the heart of the child.

Although specific embodiments have been illustrated and described, numerous modifications and variations are still possible without departing from the scope of the invention. The scope of the invention is limited by the accompanying claims.

## Claims

1. A delivery apparatus for a medical device, with the delivery apparatus (10; 10a) configured to deliver a medical device (70) to a heart defect in a heart by a catheter (90), with the delivery apparatus (10; 10a) comprising:
a first wire section (11) including a proximal end and a distal end;
a second wire section (12; 12a; 12b; 12c) including a first end (1201) and a second end (1202) opposite to the first end (1201), with the first end (1201) of the second wire section (12; 12a; 12b; 12c) securely connected to the distal end of the first wire section (11), with a connecting member (121) mounted to the second end (1202) of the second wire section (12; 12a; 12b; 12c), with the connecting member (121) adapted to be connected to the medical device (70), with the second wire section (12; 12a; 12b; 12c) having a cross sectional area smaller than a cross sectional area of the first wire section (11), wherein when the medical device (70) is delivered by the delivery apparatus (10; 10a) to the heart defect and is seated in a deployed position occluding the heart defect after the catheter (90) is retracted, the second wire section (12; 12a; 12b; 12c) is configured to have a flexibility maintaining connection with the medical device (70) to absorb interfering vibration from heartbeat, avoiding the medical device (70) from deviating from the deployed position.

2. The delivery apparatus for a medical device as claimed in claim 1, further comprising a coupling member (13) between the first wire section (11) and the second wire section (12; 12a; 12b; 12c), with the coupling member (13) riveted to the first wire section (11) and the second wire section (12; 12a; 12b; 12c) to securely connect the first end (1201) of the second wire section (12; 12a; 12b; 12c) to the distal end of the first wire section (11).

3. The delivery apparatus for a medical device as claimed in claim 1 or 2, with the first wire section (11) formed by a plurality of metal wires (111) stranded together, with the second wire section (12a) formed by at least one of the plurality of metal wires (111), and with a number of the at least one of the plurality of metal wires (111) forming the second wire section (12a) being smaller than a number of the plurality of metal wires (111) forming the first wire section (11), wherein the second wire section (12a) is formed by one of the plurality of metal wires (111).

4. The delivery apparatus for a medical device as claimed in any one of claims 1 to 3, with a virtual axis (L) extending through an axis of the first wire section (11) and an axis of the second wire section (12a), with the first wire section (11) having a maximum width (W1) perpendicular to the virtual axis (L), with the second wire section (12a) having a maximum width (W2) perpendicular to the virtual axis (L), with the maximum width (W2) of the second wire section (12a) not larger than the maximum width (W1) of the first wire section (11), with a coupling member (13) mounted between the first wire section (11) and the second wire section (12a), and with the coupling member (13) riveted to the first wire section (11) and the second wire section (12a) to securely connect the second wire section (12a) to the first wire section (11).

5. The delivery apparatus for a medical device as claimed in any one of claims 1 to 4, with the connecting member (121) including a threaded portion (122) in threading connection with the medical device (70), and with the threaded portion (122) having a twist controllability configured to permit the threaded portion (122) to rotate relative to the medical device (70) when the delivery apparatus (10) is rotated, thereby releasing the medical device (70), with the first wire section (11) formed by a plurality of metal wires (111) stranded together in a rotational direction, and with the threaded portion (122) having a thread direction in the rotational direction.

6. The delivery apparatus for a medical device as claimed in any one of claims 1 to 5, with the second wire section (12; 12a; 12b; 12c) having a length permitting the second wire section (12; 12a; 12b; 12c) to be completely located in the heart without contacting with blood vessels of the heart after the medical device (70) has been deployed to occlude the heart defect and after the catheter (90) has been retracted.

7. The delivery apparatus for a medical device as claimed in claim 6, with the second wire section (12; 12b; 12c) having circular cross sections, with a spring wire (112) coiled around the first wire section (11), and with the medical device (70) being a heart defect occluder.

8. The delivery apparatus for a medical device as claimed in claim 6, with the second wire section (12a) having non-circular cross sections, with a spring wire (112) coiled around the first wire section (11), and with the medical device (70) being a heart defect occluder.

9. The delivery apparatus for a medical device as claimed in any one of claims 1 to 3, with a virtual axis (L) extending through an axis of the first wire section (11) and an axis of the second wire section (12b), with the second wire section (12b) including a flexible portion capable of absorbing interfering vibration from the heartbeat, with the flexible portion including a first portion (123b) and a second portion (124b), with each of the first portion (123b) and the second portion (124b) extending along the virtual axis (L), with the first portion (123b) having a first end securely connected to the distal end of the first wire section (11), and with the second wire section (12b) having a first end connected to a second end of the first portion (123b) opposite to the first wire section (11).

10. The delivery apparatus for a medical device as claimed in claim 9, with the first portion (123b) having a uniform cross sectional area perpendicular to the virtual axis (L) and equal to a maximum cross sectional area of the flexible portion of the second wire section (12b), and with the second portion (124b) having a uniform cross sectional area smaller than the uniform cross sectional area of the first portion (123b).

11. The delivery apparatus for a medical device as claimed in claim 9 or 10, with the flexible portion of the second wire section (12b) having a maximum width (W2) perpendicular to the virtual axis (L), with the first portion (123b) having a maximum width (W3) perpendicular to the virtual axis (L), with the maximum width (W3) of the first portion (123b) equal to the maximum width (W2) of the flexible portion of the second wire section (12b), with the second portion (124b) having a maximum width (W4) perpendicular to the virtual axis (L), and with the maximum width (W4) of the second portion (124b) not larger than the maximum width (W3) of the first portion (123b), with the delivery apparatus for a medical device further comprising a first coupler (14b) between the first portion (123b) and the second portion (124b), with the first coupler (14b) including a first end riveted to the second end of the first portion (123b) and a second end riveted to the second portion (124b), wherein riveting of the first coupler (14b) includes:
forming two recessed portions (126b) in two diametrically opposed sides of the second end of the first portion (123b) by extrusion;
forming two first recessed portions (127b) in two diametrically opposed sides of the first end of the second portion (124b) by extrusion; and
mounting the first coupler (14b) around the second end of the first portion (123b) and the first end of the second portion (124b), with the first coupler (14b) including a first end and a second end, with two diametrically opposed sides of the first end of the first coupler (14b) being punched to form two first recessed portions (141b), with two diametrically opposed sides of the second end of the first coupler (14b) being punched to form two second recessed portions (142b), with the two first recessed portions (141b) of the first coupler (14b) received in and aligned with the two recessed portions (126b) of the first portion (123b), and with the two second recessed portions (142b) of the first coupler (14b) received in and aligned with the two first recessed portions (127b) of the second portion (124b).

12. The delivery apparatus for a medical device as claimed in any one of claims 9 to 11, with the flexible portion of the second wire section (12b) further including a third portion (125b) extending along the virtual axis (L), with the third portion (125b) including a first end connected to a second end of the second portion (124b) opposite to the first portion (123b), with the third portion (125b) having a cross sectional area smaller than a cross sectional area of the second portion (124b), with the third portion (125b) having a maximum width (W5) perpendicular to the virtual axis (L), with the maximum width (W5) of the third portion (125b) not larger than the maximum width (W4) of the second portion (124b), with a second coupler (15b) mounted between the second portion (124b) and the third portion (125b), with the second coupler (15b) including a first end riveted to the second portion (124b) and a second end riveted to the third portion (125b), wherein riveting of the second coupler (15b) includes:
forming two second recessed portions (128b) in two diametrically opposed sides of the second end of the second portion (124b) by extrusion;
forming two first recessed portions (129b) in two diametrically opposed sides of the first end of the third portion (125b) by extrusion; and
mounting the second coupler (15b) around the second end of the second portion (124b) and the first end of the third portion (125b), with the second coupler (15b) including a first end and a second end, with two diametrically opposed sides of the first end of the second coupler (15b) being punched to form two first recessed portions (151b), with two diametrically opposed sides of the second end of the second coupler (15b) being punched to form two second recessed portions (152b), with the two first recessed portions (151b) of the second coupler (15b) received in and aligned with the two second recessed portions (128b) of the second portion (124b), and with the two second recessed portions (152b) of the second coupler (15b) received in and aligned with the two first recessed portions (129b) of the third portion (125b).

13. The delivery apparatus for a medical device as claimed in claim 11 or 12, with the first coupler (14b) including a first engaging member (143b) and a second engaging member (144b), with the two first recessed portions (141b) of the first coupler (14b) formed in the first engaging member (143b) of the first coupler (14b), with the first engaging member (143b) of the first coupler (14b) securely connected to the second end of the first portion (123b), with the two second recessed portions (142b) of the first coupler (14b) formed in the second engaging member (144b) of the first coupler (14b), with the second engaging member (144b) of the first coupler (14b) securely connected to the first end of the second portion (124b), with the first and second engaging members (143b, 144b) of the first coupler (14b) fixed to each other by screwing and bonding, with the second coupler (15b) including a first engaging member (153b) and a second engaging member (154b), with the two first recessed portions (151b) of the second coupler (15b) formed in the first engaging member (153b) of the second coupler (15b), with the first engaging member (153b) of the second coupler (15b) securely connected to the second end of the second portion (124b), with the two second recessed portions (152b) of the second coupler (15b) formed in the second engaging member (154b) of the second coupler (15b), with the second engaging member (154b) of the second coupler (15b) securely connected to the first end of the third portion (125b), with the first and second engaging members (153b, 154b) of the second coupler (15b) fixed to each other by screwing and bonding.

14. The delivery apparatus for a medical device as claimed in claim 12 or 13, with the connecting member (121) riveted to a second end of the third portion (125b) opposite to the second portion (124b), wherein riveting of the connecting member (121) includes:
forming two second recessed portions (130b) in two diametrically opposed sides of the second end of the third portion (125b) by extrusion; and
mounting the connecting member (121) around the second end of the third portion (125b), with two diametrically opposed sides of the connecting member (121) being punched to form two first recessed portions (1211), and with the two first recessed portions (1211) of the connecting member (121) received in and aligned with the two second recessed portions (130b) of the third portion (125b).

15. The delivery apparatus for a medical device as claimed in any one of claims 12 to 14, wherein each of the first coupler (14c) and the second coupler (15c) is integrally formed.
